# EUROPEAN PATENT APPLICATION

(11) **EP 3 673 848 A1**
(43) Date of publication of application: **01.07.2020**
(21) Application number: 19218159.2
(22) Date of filing: 19.12.2019
(51) Int. Cl.: A61B 18/14, A61M 25/10, A61B 18/00, A61B 17/00

(54) **ABLATION BALLOON CATHETER ALLOWING BLOOD FLOW**

(30) Priority: 27.12.2018 US 201816234229
(71) Applicant: Biosense Webster (Israel) Ltd., Yokneam, 2066717 (IL)
(72) Inventor: PAPAIOANNOU, Athanassios, Irwindale, CA California 91706 (US); KEYES, Joseph Thomas, Irwindale, CA California 91706 (US)
(74) Representative: Small, Gary James

(57) **Abstract**

A balloon catheter includes a shaft, a balloon including an expandable membrane, one or more ablation elements, and one or more passages via the expandable membrane. The shaft is configured for insertion into an organ of a patient. The balloon is fitted at a distal end of the shaft. The one or more ablation elements are disposed over the expandable membrane and are configured for tissue ablation. The one or more passages via the expandable membrane are configured to allow blood to flow between a distal end and a proximal end of the balloon, while the balloon is in an expanded configuration.

## Description

### FIELD OF THE INVENTION

The present invention relates generally to medical probes, and particularly to ablation balloon catheters.

### BACKGROUND OF THE INVENTION

Various known catheter designs have an inflatable balloon fitted at their distal end. For example, U.S. Patent 6,905,494 describes an enhanced method and device to treat atrial fibrillation or inhibit or reduce restenosis following angioplasty or stent placement. A cryoablation balloon catheter is provided for treating atrial fibrillation or inhibit or reduce restenosis following angioplasty or stent placement. A balloon-tipped catheter is disposed in the area treated or opened through balloon angioplasty immediately following angioplasty. A cooling fluid can also flow into the balloon to freeze the tissue adjacent the balloon, this cooling being associated with reduction of restenosis. A similar catheter may be used to reduce atrial fibrillation by inserting and inflating the balloon such that an exterior surface of the balloon contacts at least a partial circumference of the portion of the pulmonary vein adjacent the left atrium. In an embodiment, the cryoablation balloon catheter may be employed to perform cryoablation while allowing blood flow during an ablation procedure.

As another example, U.S. Patent 6,540,734 describes a multi-lumen balloon for use in a fluted balloon centering catheter. The multi-lumen balloon maintains a radiation source at the center of a cardiovascular artery, has improved blood perfusion capability, and has improved balloon refolding characteristics. The method of fabricating a multi-lumen balloon designed for a radiation centering catheter uses an improved extrusion process that allows the manufacture of the multi-lumen balloon subassembly to be done separately from the catheter shaft assembly.

U.S. Patent 5,342,301 describes multi-lumen dilatation balloons for use as or in conjunction with balloon dilation catheters, and methods for making such balloons. In an embodiment, a balloon is formed as a multi-lumen balloon, in accordance with the present invention, for providing the necessary blood flow when the balloon is inflated, and for providing the necessary inflation so as to achieve dilatation of the blood vessel.

7,563,247 describes a renal flow system and method to direct fluid into renal arteries from a location within the aorta. A renal flow assembly has a tapered tube that is adjustable between a radially collapsed condition for delivery to the location through a delivery sheath and a radially expanded condition that divides aortic flow into exterior and interior paths. The tube's wall is made from a sheet of flexible material. Two metal rings radially support the tube's ends and are connected by a longitudinal spine support. A fluid delivery assembly injects drug to flow along the exterior flow path and the tubular wall directs the agent into the renal artery ostium. The flow assembly allows an interventional device, e.g., a delivery catheter, to advance across the location while directing blood into the kidneys and perfusing downstream circulation. The renal flow assembly and distal device are used within a common guide sheath.

### SUMMARY OF THE DISCLOSURE

An embodiment of the present invention provides a balloon catheter including a shaft, a balloon including an expandable membrane, one or more ablation elements, and one or more passages via the expandable membrane. The shaft is configured for insertion into an organ of a patient. The balloon is fitted at a distal end of the shaft. The one or more ablation elements are disposed over the expandable membrane and are configured for tissue ablation. The one or more passages via the expandable membrane are configured to allow blood to flow between a distal end and a proximal end of the balloon, while the balloon is in an expanded configuration.

In some embodiments, the one or more passages via the expandable membrane are configured to deliver fluid distally through the balloon.

In some embodiments, the delivered fluid includes at least one of saline solution and a medication.

In an embodiment, the one or more passages include internal lumens in a wall of the expandable membrane.

In an embodiment, the one or more passages include external passages via the expandable membrane.

In some embodiments, the one or more passages are configured to allow blood to flow simultaneously with applying ablation using the ablation elements.

There is additionally provided, in accordance with an embodiment of the present invention, a method for manufacturing a balloon catheter, the method including fitting, at a distal end of a shaft for insertion into an organ of a patient, a balloon including an expandable membrane, wherein the balloon includes a distal end and a proximal end. One or more ablation elements are disposed over the expandable membrane and are configured for tissue ablation. One or more passages are formed via the expandable membrane, wherein the one or more passages are configured to allow blood to flow between the distal end and the proximal end of the balloon, while the balloon is in an expanded configuration.

In some embodiments, forming the passages includes forming internal lumens in a wall of the expandable membrane.

In an embodiment, forming the internal lumens includes (a) co-extruding the internal lumens and the expandable membrane, (b) inserting respective mandrels in the lumens during co-extrusion of the lumens, and (c) terminating the internal lumens and exposing proximal and the and distal ends of the lumens.

In another embodiment, forming the passages includes thermally welding lumens to the membrane.

In an embodiment, forming the passages includes forming external passages via the expandable membrane.

There is further provided, in accordance with an embodiment of the present invention, a method, including inserting into an organ of a patient a balloon catheter comprising a shaft and a balloon fitted at a distal end of the shaft, the balloon including an expandable membrane, one or more ablation elements disposed over the expandable membrane and are configured for tissue ablation, and one or more passages formed via the expandable membrane. The balloon is expanded to an expanded configuration. While the balloon is in the expanded configuration, tissue in the organ is ablated using the ablation elements for ablating tissue while allowing blood to flow between a distal end and a proximal end of the balloon via the passages.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based position-tracking and ablation system comprising a radiofrequency (RF) ablation balloon, in accordance with an embodiment of the present invention;
Figs. 2A and 2B are schematic side and frontal equatorial cross-sectional views, respectively, of a balloon catheter, in accordance with an embodiment of the present invention; and
Figs. 3A and 3B are a front view and a volume rendering, respectively, of a balloon catheter, in accordance with another embodiment of the present invention.

### DETAILED DESCRIPTION OF EMBODIMENTS

### OVERVIEW

An expandable ablation balloon, such as a balloon for performing radiofrequency (RF) ablation, cryoablation, or other types of tissue ablation, may be fitted at the distal end of a catheter that is navigated through the vascular system and inserted into an organ for performing ablation treatment. For example, the balloon catheter may be inserted through the cardiovascular system into a heart, for ablating the ostium of a pulmonary vein (PV) in a treatment of arrhythmia known as pulmonary vein isolation (PVI). A balloon catheter may be used in the way described above because it enables ablation of tissue simultaneously over the entire circumference of a blood vessel, such as a PV, a procedure which is clinically advantageous.

To implement such a simultaneous full-circumference ablation, the balloon may be inserted such that, after being expanded, it contacts the entire periphery of blood vessel tissue, such as the ostium of a PV. If a physical contact is achieved over the entire balloon circumference, there is consequently virtually complete occlusion of the targeted PV by the balloon. A complete occlusion, however, is undesirable, since it may lead to compromised blood hemodynamics distally to the balloon, inducing blood stagnation (i.e., no flow) within the occluded PV. Stagnation of blood, added to increased temperature due to, for example, RF ablation, may increase the chance for thrombus formation.

Embodiments of the present invention that are described hereinafter provide ablation balloon catheters that are configured to allow blood to flow through the ablated blood vessel (e.g., a PV) during the ablation. The disclosed balloons include one or more passages through which the blood in the blood vessel can flow while the balloon is in the expanded configuration. For example, during a PVI procedure, the passages allow blood to flow from the PV, proximally into the left atrium, through the expanded balloon that is in contact with the perimeter of the PV.

In some embodiments, the disclosed balloon incorporates one or more pass-through ancillary lumens that run tangentially and interiorly to its surface. In an embodiment, the disclosed balloon has one or more lumens in a wall of a membrane of the balloon, which are implemented as a coextrusion of the lumens, with the main lumen forming the balloon membrane. Mandrels are inserted in the core of the one or more additional lumens to keep them open during the extrusion (i.e., blowing and stretching) process that forms the balloon. The lumens are then terminated (either mechanically, with a laser, or by another suitable technique) and exposed at the two ends (proximal and distal) of the lumens to allow blood to enter and to exit the occluding balloon.

In some embodiments, the one or more coextruded lumens are attached either interiorly or exteriorly along the surface of the balloon membrane. In an optional embodiment, the lumens are added after forming the balloon, and, assuming thermal compatibility between the balloon and lumen materials, the lumens are thermally welded onto the balloon surface.

Alternatively, the ancillary lumens could be located fully interiorly to the balloon (i.e. "floating" inside the balloon and terminated and exposed at the proximal and distal ends of the balloon).

In an alternative embodiment, one or more external passages, such as made of lobed-design or pleats via the balloon membrane are formed in the balloon. The pleats allow blood to pass, but the balloon can still be used for ablating tissue using ablation elements, such as RF electrodes disposed over the un-pleated area, as described below.

The disclosed solutions allow the flow of blood during balloon ablation of a blood vessel. The proposed designs facilitate blood hemodynamics at the vicinity of the ablated area, with consequential minimization of blood stagnation and potential reduction of probability of thrombus creation. The disclosed balloon may thus allow patients who were previously precluded from undergoing a balloon ablation procedure, for example, due to risk of stroke, to receive balloon ablation treatment.

### SYSTEM DESCRIPTION

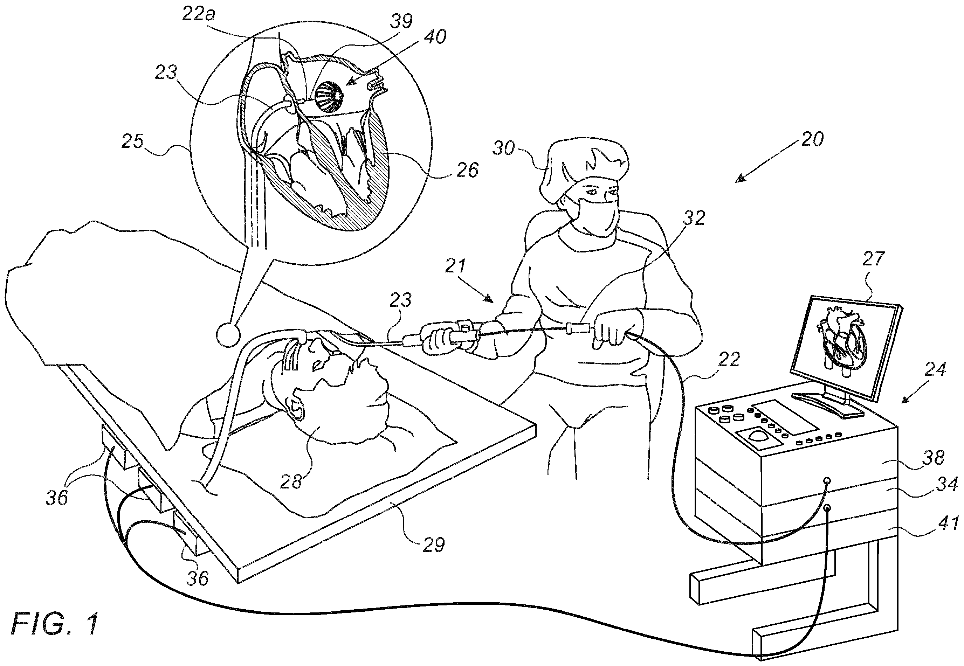

Fig. 1 is a schematic, pictorial illustration of a catheter-based position-tracking and ablation system 20 comprising an RF ablation balloon 40, in accordance with an embodiment of the present invention. System 20 comprises a catheter 21, wherein, as seen in inset 25, a distal end 22a of shaft 22 of catheter 21 is inserted through a sheath 23 into a heart 26 of a patient 28 lying on a table 29. As further shown in inset 25, distal end 22a comprises a magnetic sensor 39, contained within distal end 22a just proximally to balloon 40.

The proximal end of catheter 21 is connected to a control console 24. In the embodiment described herein, catheter 21 may be used for any suitable therapeutic and/or diagnostic purpose, such as electrical sensing and/or ablation of tissue in heart 26.

During navigation of distal end 22a in heart 26, console 24 receives signals from magnetic sensor 39 in response to magnetic fields from external field generators 36, for example, for the purpose of measuring the position of ablation balloon 40 in the heart and, optionally, presenting the tracked position on a display 27. Magnetic field generators 36 are placed at known positions external to patient 28, e.g., below patient table 29. Console 24 also comprises a driver circuit 34, configured to drive magnetic field generators 36.

In an embodiment, position signals received from position sensor 39 are indicative of the position of ablation balloon 40 in the coordinate system of position tracking and ablation system 20. The method of position sensing using external magnetic fields is implemented in various medical applications, for example, in the CARTO™ system, produced by Biosense-Webster Inc. (Irvine, California), and is described in detail in U.S. Patents 5,391,199 and 6,332,089, and in PCT Patent Publication WO 96/05768, whose disclosures are all incorporated herein by reference.

Physician 30 navigates the distal end of shaft 22 to a target location in heart 26 by manipulating shaft 22 using a manipulator 32 near the proximal end of the catheter and/or deflection from the sheath 23. During the insertion of shaft 22, balloon 40 is maintained in a collapsed configuration by sheath 23. By containing balloon 40 in a collapsed configuration, sheath 23 also serves to minimize vascular trauma along the way to target location.

Control console 24 comprises a processor 41, typically a general-purpose computer, with suitable front end and interface circuits 38 for receiving signals from catheter 21, as well as for applying treatment via catheter 21 in heart 26 and for controlling the other components of system 20. Processor 41 typically comprises a general-purpose computer with software programmed to carry out the functions described herein. The software may be downloaded to the computer in electronic form, over a network, for example, or it may, alternatively or additionally, be provided and/or stored on non-transitory tangible media, such as magnetic, optical, or electronic memory.

The example configuration shown in Fig. 1 is chosen purely for the sake of conceptual clarity. The disclosed techniques may similarly be applied using other system components and settings. For example, system 20 may comprise other components and perform non-cardiac ablative treatments.

### RADIOFREQUENCY (RF) ABLATION BALLOON CATHETER ALLOWING BLOOD FLOW

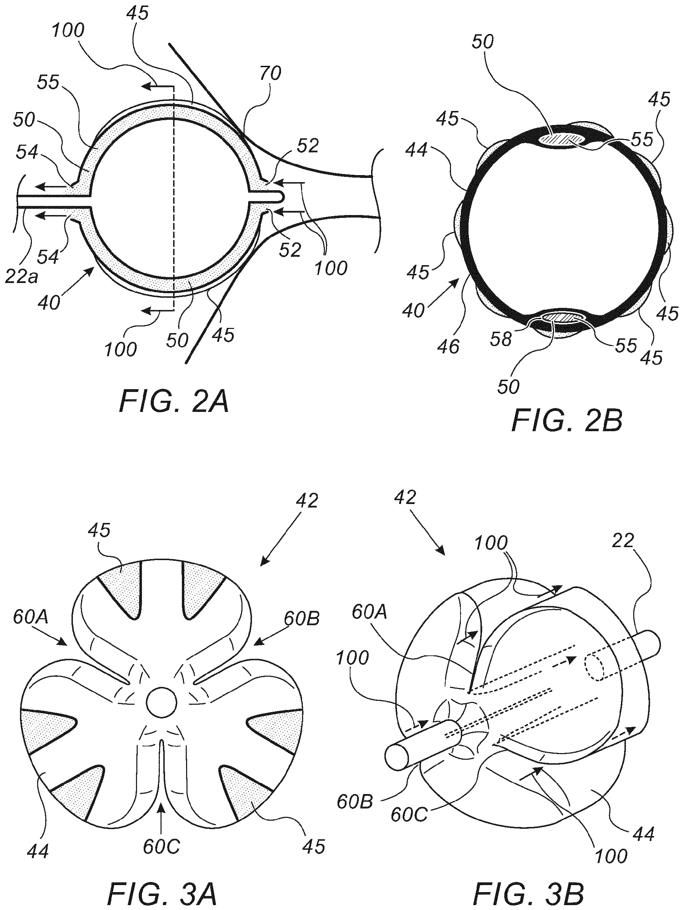

Figs. 2A and 2B are schematic side and frontal equatorial cross-sectional views, respectively, of balloon catheter 40, in accordance with an embodiment of the present invention. Fig. 2A shows a longitudinal cross-section of expanded balloon 40 occluding an ostium 70 of a blood vessel, such as an ostium of a PV. As seen, RF ablation electrodes 45 are disposed over the exterior of balloon 40, where electrodes 45 are pressed between balloon 40 and the tissue of ostium 70.

As further seen, blood 55 enters lumens 50 through terminated distal ends 52 and flows proximally through lumens 50 to be released at terminated proximal ends 54 of lumens 50. Direction of blood flow is indicated by arrows 100.

Fig. 2B shows an equatorial cross-section, where lumens 50 are co-extrusions in an expanded membrane 44 of balloon 40. As seen, blood 55 flows in a non-circular shaped lumen 50 or passageway that is formed in the wall of membrane 44. The passageway 50 is formed on the circumferential wall 46 of membrane 44, and specifically on the inner circumferential surface of the wall 46 of the membrane 44. To keep the passageway 50 from collapsing when the membrane 44 is expanded, mandrel(s) can be provided in each passageway. Mandrels 58, which are made of a less flexible (i.e., stiffer) material, such as PTFE (i.e., Teflon) and PTFE-coated steel, provide structural support that holds lumens 50 open while membrane 44 is in an expanded form. While the example of Fig. 2B shows two passageways 50 with respective mandrels 58, it is within the scope of this invention to provide more than two passageways.

As further seen in Fig. 2B, the disclosed balloon configuration has the RF ablation electrodes 45 disposed over the exterior of membrane 44, over an entire circumference of balloon 40, which enables a simultaneous ablation along an entire perimeter of ostium 70, while pulmonary circulation (i.e., pulmonary blood flow) continues through lumens 50.

Figs. 3A and 3B are a schematic pictorial illustration and a volume rendering, respectively, of a balloon catheter 42, in accordance with another embodiment of the present invention. Balloon 42 may be used, for example, instead of balloon 40 in system 20 of Fig. 1.

Fig. 3A is frontal illustration of balloon 42 in an expanded form. As seen in the figure, balloon 42 has pleats 60A-C, incorporated in membrane 44, to allow blood flow through a circumferential surface of the membrane 44 and specifically the outer circumferential surface of membrane 44. In an embodiment, the pleats are made while manufacturing the balloon. An inflated balloon membrane is introduced into a "pleating station" in which "pleating dies" (usually heated) encircle the balloon so as to form the pleats. After appropriate temperature and time exposure to these forming dies, vacuum is applied to the balloon to ensure retaining the pleating. As seen, electrodes 45 are disposed on the exterior of membrane 44, on un-pleated areas, to allow the simultaneous RF ablation and blood flow. Direction of blood flow is indicated in Fig. 3B by arrows 100.

The illustrations shown in Figs. 2A and 2B, and in Figs. 3A and 3B, are chosen purely for the sake of conceptual clarity. For example, the disclosed manufacturing elements, such as mandrels 58, are brought by way of example and were chosen purely for the sake of conceptual clarity. In alternative embodiments, manufacturing elements and manufacturing methods may substantially differ from that presented in this highly simplified manner. In the balloon of Figs. 2A and 2B, for example, other lumen shapes, such as lumens having a cross-section other than the depicted oval, are possible. Also, the number of lumens may vary (i.e., one or more lumens may be formed). In the balloon of Figs. 3A and 3B, for example, the number of pleats may be higher, e.g., having a pleat between each two electrodes. Additionally or alternatively, the pleats may be shallower than seen.

Although the embodiments described herein mainly address design and manufacturing of cardiac catheters, the methods and systems described herein can also be used in other applications, such as in otolaryngology or neurology procedures. Additionally, the disclosed technique may be used, inter alia, in facilitating distally flushing an otherwise blocked blood vessel, such as PV, with fluid, such as saline solution and/or medication, during "peri-ablation" time (just before, during and just after ablation). For example, the flushing may be achieved by adding a dedicated working channel and a lumen to the disclosed balloon catheter in fluid communication with the balloon's channels for flushing.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A balloon catheter, comprising:
a shaft configured for insertion into an organ of a patient;
a balloon comprising an expandable membrane, which is fitted at a distal end of the shaft;
one or more ablation elements, which are disposed over the expandable membrane and are configured for tissue ablation; and
one or more passages proximate a circumferential wall of the expandable membrane, wherein the one or more passages are configured to allow blood to flow between a distal end and a proximal end of the balloon, while the balloon is in an expanded configuration.

2. The balloon catheter according to claim 1, wherein the one or more passages via the expandable membrane are configured to deliver fluid distally through the balloon.

3. The balloon catheter according to claim 2, wherein the delivered fluid comprises at least one of saline solution and a medication.

4. The balloon catheter according to claim 1, wherein the one or more passages comprise internal lumens in a wall of the expandable membrane.

5. The balloon catheter according to claim 1, wherein the one or more passages comprise external passages via the expandable membrane.

6. The balloon catheter according to claim 1, wherein the one or more passages are configured to allow blood to flow simultaneously with applying ablation using the ablation elements.

7. A method for manufacturing a balloon catheter, the method comprising:
fitting, at a distal end of a shaft for insertion into an organ of a patient, a balloon comprising an expandable membrane, wherein the balloon comprises a distal end and a proximal end;
disposing over the expandable membrane one or more ablation elements, which are configured for tissue ablation; and
forming one or more passages via a circumferential surface of the expandable membrane, wherein the one or more passages are configured to allow blood to flow between the distal end and the proximal end of the balloon, while the balloon is in an expanded configuration.

8. The method according to claim 7, wherein forming the passages comprises forming internal lumens in an inner wall of the expandable membrane.

9. The method according to claim 8, wherein forming the internal lumens comprises:
co-extruding the internal lumens and the expandable membrane;
inserting respective mandrels in the lumens during co-extrusion of the lumens; and
terminating the internal lumens and exposing proximal and the and distal ends of the lumens.

10. The method according to claim 7, wherein forming the passages comprises thermally welding lumens to the membrane.

11. The method according to claim 7, wherein forming the passages comprises forming external passages via the expandable membrane.

12. A method, comprising:
inserting into an organ of a patient a balloon catheter comprising a shaft and a balloon fitted at a distal end of the shaft, the balloon comprising an expandable membrane, one or more ablation elements disposed over the expandable membrane and are configured for tissue ablation, and one or more passages formed via a circumferential surface of the expandable membrane;
expanding the balloon to an expanded configuration; and
while the balloon is in the expanded configuration, ablating tissue in the organ using the ablation elements for ablating tissue while allowing blood to flow between a distal end and a proximal end of the balloon via the passages.

13. The method according to claim 12, further comprising delivering fluid distally through the balloon via the one or more passages.

14. The method according to claim 13, wherein delivering the fluid comprises delivering at least one of saline solution and a medication.
